# EUROPÄISCHE PATENTANMELDUNG

(11) **EP 4 817 410 A1**
(43) Veröffentlichungstag der Anmeldung: **07.10.2026**
(21) Anmeldenummer: 26153307.9
(22) Anmeldetag: 21.01.2026
(51) Int. Cl.: A01K 5/00, A01K 5/02

(54) **FUTTERMISCHWAGEN UND VERFAHREN ZUM HERSTELLEN EINES FUTTERMITTELGEMISCHES**

(30) Priorität: 02.04.2025 AT 502332025
(71) Anmelder: Wasserbauer Holding GmbH, 4595 Waldneukirchen (AT)
(72) Erfinder: Wasserbauer, Franz, 4595 Waldneukirchen (AT)
(74) Vertreter: KLIMENT & HENHAPEL

(57) **Zusammenfassung**

Fahrbarer Futtermischwagen (2) zur Verteilung eines Futtermittelgemisches in landwirtschaftlichen Betrieben mit einem mit Futtermittelkomponenten befüllbaren Futtermittelbehälter (4), in dem ein Futtermischer (8) zur Vermengung der Futtermittelkomponenten zum Futtermittelgemisch angeordnet ist, wobei der Futtermischwagen (2) mit einer Abgabevorrichtung (5) zur Abgabe des Futtermittelgemisches während einer Fütterungsfahrt versehen ist, und eine Steuereinrichtung (11) zur Steuerung der Befüllung mit den Futtermittelkomponenten in einer Befüllstation (6) vorgesehen ist. Hierbei wird vorgeschlagen, dass im Futtermittelbehälter (4) zumindest ein Feuchtigkeitssensor (14) zur Messung der Feuchtigkeit des Futtermittelgemisches angeordnet ist, der mit der Steuereinrichtung (11) verbunden ist.

## Beschreibung

Die Erfindung bezieht sich auf einen fahrbaren Futtermischwagen zur Verteilung eines Futtermittelgemisches in landwirtschaftlichen Betrieben mit einem mit Futtermittelkomponenten befüllbaren Futtermittelbehälter, in dem ein Futtermischer zur Vermengung der Futtermittelkomponenten zum Futtermittelgemisch angeordnet ist, wobei der Futtermischwagen mit einer Abgabevorrichtung zur Abgabe des Futtermittelgemisches während einer Fütterungsfahrt versehen ist, und eine Steuereinrichtung zur Steuerung der Befüllung mit den Futtermittelkomponenten in einer Befüllstation vorgesehen ist, gemäß dem Oberbegriff von Anspruch 1. Die Erfindung bezieht sich ferner auf ein Verfahren zum Herstellen eines Futtermittelgemisches aus Futtermittelkomponenten in einem mit den Futtermittelkomponenten befüllbaren Futtermittelbehälter eines fahrbaren und zur Abgabe des Futtermittelgemisches während einer Fütterungsfahrt ausgelegten Futtermischwagens, gemäß dem Oberbegriff von Anspruch 5.

Fahrbare Futtermischwagen werden in der modernen Stalltechnik beispielsweise in Milchwirtschaftsbetrieben oder in Rindermastbetrieben eingesetzt, um Futtergemische aus mehreren Futtermittelkomponenten abzumischen und im Futtergang entlang eines Barrens auszubringen. Das Vieh befindet sich auf der anderen Seite des Barrens, der aber so ausgeführt ist, dass das Vieh das im Futtergang ausgebrachte Futtergemisch zur Futteraufnahme erreichen kann. Das Futter sollte dabei gelockert und gut gemischt dem Vieh angeboten werden. Hierbei ist es vorteilhaft mehrmals täglich kleinere Futtermengen auszubringen, um stets eine frische Futtervorlage zu gewährleisten. Das Ausdosieren kleinerer Futtermengen unterstützt das natürliche Fressverhalten der Tiere und sorgt damit für eine effizientere Futterverwertung, was für die Tiergesundheit und damit auch die Produktivität des Betriebes förderlich ist. Der Viehbestand eines Betriebes unterteilt sich außerdem zumeist in unterschiedliche Leistungsgruppen mit unterschiedlichem Bedarf an Futtermenge und Futterzusammensetzung. Es ist somit mehrmals täglich Futter in unterschiedlichen Mengen und unterschiedlicher Zusammensetzung auszubringen, was mit einem entsprechenden Arbeitsaufwand verbunden ist.

Fahrbare Futtermischwagen reduzieren diesen Arbeitsaufwand, indem sie als autonome Fahrzeuge ausgeführt sind, die Futtermittelkomponenten in einer Befüllstation aufnehmen, abmischen und entlang des Futterganges ausbringen. Sie werden von einer Steuereinrichtung gesteuert und absolvieren eine vorprogrammierte Fahrstrecke, indem sie sich beispielsweise anhand von Markierungsmagneten orientieren, die im Stallboden angeordnet sind. Die Steuereinrichtung ist zumeist am Futtermischwagen selbst angeordnet, wo über ein entsprechendes Display auch die Dateneingabe und Datenausgabe sowie eine Konfigurierung der Steuereinrichtung erfolgen kann. Es wäre aber auch denkbar die Steuereinrichtung als externe Einheit auszuführen, die auf einem PC, einem Tablet oder auch einem Mobilfunkgerät ("Smartphone") installiert sein kann. Über die Steuereinrichtung können insbesondere die Zusammensetzung des Futtermittelgemisches, die Menge, die Fütterungszeiten und die Fahrtrouten festgelegt werden. Im täglichen Betrieb steuert die Steuereinrichtung den Futtermischwagen je nach programmierter Uhrzeit zur Befüllstation, in der die einzelnen Futtermittelkomponenten des Grundfutters, beispielsweise Grassilage, Heu oder Maissilage, sowie die Futtermittelkomponenten des Kraftfutters wie Getreide- oder industriell hergestellte Mischfutterkomponenten, bereitgehalten werden. Für die Art der Lagerhaltung von Futtermittelkomponenten und deren Abfüllung in den Futtermischwagen sind in der Praxis unterschiedliche Ausführungen bekannt. Der Futtermischwagen fährt beispielsweise einzelne Vorratsbehälter der Befüllstation ab, die auch als "Ports" bezeichnet werden, und wird dort mit den einzelnen Futtermittelkomponenten befüllt. Zusätzlich kann auch eine Befüllung über Förderbänder oder über Gebläse aus externen Fahrsilos und dergleichen vorgesehen sein. Zudem ist auch eine Befüllung über Kräne und dergleichen oder über stationäre Mischanlagen möglich.

Der Futtermischwagen verfügt über einen Futtermittelbehälter, der beispielsweise als Vertikalmischbehälter ausgeführt ist und durch Einwurf von oben befüllt werden kann. Im Futtermittelbehälter ist ein Futtermischer zur Vermengung der Futtermittelkomponenten zum Futtermittelgemisch angeordnet, der beispielsweise als Vertikalmischer mit vertikaler Drehachse, etwa in Form einer Mischschnecke, ausgeführt ist. Der Vertikalmischer kann auch so ausgeführt sein, dass er das Futter zusätzlich zerkleinert, indem er entsprechende Schneidorgane aufweist. Die einzelnen Befüllmengen können in der Steuereinrichtung festgelegt werden, die die einzelnen Vorratsbehälter und deren Auswurfeinrichtungen in der Befüllstation entsprechend ansteuert. Zumeist weist der Futtermischwagen auch eine Wiegeeinrichtung auf, mit der die Futtermittelmasse im Futtermittelbehälter gemessen werden kann. Sobald alle Futtermittelkomponenten für die betreffende Fütterungsfahrt vom Futtermischwagen aufgenommen wurden, wird mit der vorprogrammierten Fahrstrecke für diese Fütterungsfahrt begonnen. Gegebenenfalls kann zuvor noch eine befahrbare Ladestation angefahren werden, in der die Batterie des vorzugsweise elektrisch betriebenen Futtermischwagens aufgeladen wird und der Futtermischer während des Aufladevorganges bereits für eine gründliche Durchmischung der Futtermittelkomponenten im Futtermittelbehälter sorgt. Sobald die für diese Fütterungsfahrt vorgesehene Futtergasse erreicht wurde, wird eine Abgabevorrichtung des Futtermischwagens betätigt, sodass Futter ausgeworfen wird. Die Auswurfgeschwindigkeit und die Fahrgeschwindigkeit bestimmen dabei die lokal ausgebrachte Futtermenge. Der Futtermischwagen kann zudem in seinem bodennahen Bereich mit einer Schieberschürze versehen sein, die zuvor ausgebrachtes und vom Vieh während der Nahrungsaufnahme im Futtergang verstreutes Futter wieder zum Barren schiebt. Nach Absolvierung der Fütterungsfahrt und dem Ausbringen der vorgesehenen Futtermengen kehrt der Futtermischwagen wieder in eine Parkposition zurück.

Zusätzlich zur Fütterungsfahrt kann der Futtermischwagen auch so genannte Anschiebefahrten durchführen, bei denen kein Futter ausgebracht wird, sondern lediglich zuvor ausgebrachtes und vom Vieh während der Nahrungsaufnahme im Futtergang verstreutes Futter wieder zum Barren geschoben wird. Die hierfür vorgesehene Schieberschürze kann dazu auch höhenverstellbar ausgeführt sein, um beispielsweise bei Hofüberfahrten die Schieberschürze anheben zu können und bei der Fahrt entlang des Futterganges wieder absenken zu können.

Der Vorteil dieser autonom fahrenden Futtermischwagen besteht darin, dass für jede Leistungsgruppe abgestimmte Rezepturen an Futtermittelgemischen mehrmals täglich in variierenden Mengen hergestellt und zielgerecht ausgebracht werden können. Der Fütterungsvorgang verläuft dabei weitestgehend automatisch und mit minimalem Arbeitsaufwand. Der Fütterungsvorgang ist auch sehr flexibel, weil Futtermittelmengen und -zusammensetzung über die Steuereinrichtung jederzeit angepasst werden können.

Das mehrmals täglich in kleineren Futtermengen frisch ausgebrachte Futter wird vom Vieh in der Regel sehr gut angenommen. Allerdings zeigt sich, dass das Vieh in der Aufnahme von Futter durchaus wählerisch sein kann, indem es im Futter nach besonders schmackhaften Futtermittelkomponenten sucht und die anderen Futtermittelkomponenten dabei durchwühlt. Durch dieses Selektieren wird das ausgebrachte Futter vom Vieh im Futtergang verstreut, wodurch Anteile des ausgebrachten Futters für das Vieh nicht mehr erreichbar sind. Die Aufnahme des auf das jeweilige Vieh abgestimmten Futtermittels wird dadurch in Menge und Zusammensetzung beeinträchtigt.

Es besteht daher das Ziel der Erfindung darin die Aufnahme des auf das jeweilige Vieh hinsichtlich Menge und Zusammensetzung abgestimmten Futtermittels zu verbessern und dadurch die Tiergesundheit und damit auch die Produktivität des Betriebes zu verbessern.

Dieses Ziel wird durch die Merkmale von Anspruch 1 erreicht. Anspruch 1 bezieht sich auf einen fahrbaren Futtermischwagen zur Verteilung eines Futtermittelgemisches in landwirtschaftlichen Betrieben mit einem mit Futtermittelkomponenten befüllbaren Futtermittelbehälter, in dem ein Futtermischer zur Vermengung der Futtermittelkomponenten zum Futtermittelgemisch angeordnet ist, wobei der Futtermischwagen mit einer Abgabevorrichtung zur Abgabe des Futtermittelgemisches während einer Fütterungsfahrt versehen ist, und eine Steuereinrichtung zur Steuerung der Befüllung mit den Futtermittelkomponenten in einer Befüllstation vorgesehen ist. Erfindungsgemäß wird hierbei vorgeschlagen, dass im Futtermittelbehälter zumindest ein Feuchtigkeitssensor zur Messung der Feuchtigkeit des Futtermittelgemisches angeordnet ist, der mit der Steuereinrichtung verbunden ist. Einzelne Futtermittelkomponenten, insbesondere Silage- und Heukomponenten, können nämlich in ihrer Feuchtigkeit variieren, wodurch auch die Feuchtigkeit des Futtermittelgemisches variiert. Dabei handelt es sich um die Materialfeuchte, also die als freies Wasser im Futtermittel enthaltene Wassermenge. Durch den im Futtermittelbehälter angeordneten Feuchtigkeitssensor kann die Feuchtigkeit des Futtermittelgemisches gemessen werden. Der gemessene Wert wird der Steuereinrichtung zur Steuerung der Befüllung übermittelt. Die Feuchtigkeit des Futtermittelgemisches kann auf diese Weise überwacht und in weiterer Folge in einem optimalen Sollbereich gehalten werden. Bei zu geringer Feuchtigkeit des Futtermittelgemisches ist das Futtermittelgemisch zu trocken und kann durch das Vieh im Zuge der Nahrungsaufnahme leichter durchwühlt und verstreut werden. Eine optimale Feuchtigkeit stellt eine ausreichende Verklumpung des Futtermittelgemisches sicher, die dem Vieh das Selektieren erschwert und das Verstreuen des ausgebrachten Futters im Zuge der Futteraufnahme verringert. Auf diese Weise werden die Aufnahme des auf das jeweilige Vieh hinsichtlich Menge und Zusammensetzung abgestimmten Futtermittels verbessert und die Tiergesundheit und damit auch die Produktivität des Betriebes gefördert.

Vorzugsweise ist der Feuchtigkeitssensor an einer Innenwand des Futtermittelbehälters in einer unteren Hälfte des Futtermittelbehälters befestigt, damit der Feuchtigkeitssensor vom eingefüllten Futtermittel gut bedeckt ist und zuverlässige Messungen durchgeführt werden können. Zudem kann vorgesehen sein, dass zumindest zwei Feuchtigkeitssensoren vorgesehen sind, die an einer Innenwand des Futtermittelbehälters in einer unteren Hälfte des Futtermittelbehälters in derselben Höhe und entlang eines inneren Umfanges der Innenwand verteilt befestigt sind. Die Feuchtigkeitssensoren messen die Feuchtigkeit und übermitteln die gemessenen Werte an die Steuereinrichtung, wo beispielsweise eine Mittelung der gemessenen Werte vorgenommen werden kann. Die Feuchtigkeitssensoren messen beispielsweise die Leitfähigkeit oder die elektrische Kapazität der Futtermenge. Über eine anfängliche Kalibrierung können die Messwerte auf die Feuchtigkeit umgerechnet werden. Der Feuchtigkeitssensor liefert somit im praktischen Einsatz rasch verfügbare und genaue Daten zur Feuchtigkeit des Futtermittelgemisches.

Für den erfindungsgemäßen Futtermischwagen wird eine Anordnung zur Herstellung und Verteilung eines aus Futtermittelkomponenten gebildeten Futtermittelgemisches in landwirtschaftlichen Betrieben mit einem mit den Futtermittelkomponenten befüllbaren und einer Steuereinrichtung versehenen Futtermischwagen, sowie einer Befüllstation zur Befüllung des Futtermischwagens mit den Futtermittelkomponenten vorgeschlagen, wobei die Steuereinrichtung ausgelegt ist die Befüllung des Futtermischwagens mit den Futtermittelkomponenten sowie die Beimengung von Wasser in den Futtermischwagen in Abhängigkeit von einem im Futtermischwagen gemessenen Feuchtigkeitswert des Futtermittelgemisches zu steuern, wie in weiterer Folge noch genauer erläutert wird.

Hierfür wird ein entsprechendes Verfahren zum Herstellen eines Futtermittelgemisches aus Futtermittelkomponenten in einem mit den Futtermittelkomponenten befüllbaren Futtermittelbehälter eines fahrbaren und zur Abgabe des Futtermittelgemisches während einer Fütterungsfahrt ausgelegten Futtermischwagens vorgeschlagen, bei dem erfindungsgemäß vorgesehen ist, dass die Feuchtigkeit einer Futtermittelkomponente oder des Futtermittelgemisches gemessen wird, und bei Unterschreiten eines unteren Grenzwerts der gemessenen Feuchtigkeit Wasser der Futtermittelkomponente oder dem Futtermittelgemisch beigemengt wird. Bei Überschreiten eines oberen Grenzwerts der gemessenen Feuchtigkeit kann die Befüllmenge von zumindest einer beigemengten Futtermittelkomponente erhöht werden. Bei der Feuchtigkeit handelt es sich wiederum um die Materialfeuchte, also die als freies Wasser im Futtermittel enthaltene Wassermenge. Die Materialfeuchte kann mathematisch entweder über den Feuchtegehalt oder den Feuchteanteil dargestellt werden. Der Feuchtegehalt ist das Verhältnis der Masse des im Futtermittelgemisch enthaltenen Wassers zur Masse des wasserfreien Futtermittelgemisches. Der Feuchteanteil ist hingegen das Verhältnis der Masse des im Futtermittelgemisch enthaltenen Wassers zur Gesamtmasse des Futtermittelgemisches. Im Rahmen der vorliegenden Erfindung wird die Materialfeuchte vorzugsweise als Feuchteanteil dargestellt, weil die Gesamtmasse des feuchten Futtermittelgemisches einfacher bestimmt werden kann, beispielsweise über die Wiegeeinrichtung des Futtermittelwagens. Über eine kalibrierte Messung des Feuchteanteils über den Feuchtigkeitssensor kann in weiterer Folge auch die im Futtermittelgemisch enthaltene Wassermenge errechnet werden.

Der zuvor erwähnte untere Grenzwert und der obere Grenzwert legen den Sollbereich für die Feuchtigkeit des Futtermittelgemisches fest. Freilich kann es sich bei dem unteren Grenzwert und dem oberen Grenzwert auch um denselben Wert im Sinne eines Sollwertes handeln. Der Sollwert kann beispielsweise 38% Feuchteanteil betragen. Der untere Grenzwert und der obere Grenzwert zur Festlegung eines Sollbereiches können beispielsweise durch eine Abweichung von 10% vom genannten Sollwert festgelegt sein. Bei Unterschreiten eines unteren Grenzwerts der gemessenen Feuchtigkeit wird dem Futtermittelgemisch im Futtermittelbehälter Wasser beigemengt. Die Menge des beizumengenden Wassers zum Erreichen eines vorgegebenen Sollwerts in Abhängigkeit von beispielsweise einem gemessenen Feuchteanteil kann über zuvor erstellte Kalibrierkurven ermittelt werden, die in der Regel nichtlinear sind. Es erweist sich nämlich, dass bei zunehmender Trockenheit des Futtermittelgemisches eine höhere Wassermenge beigemengt werden muss um eine bestimmte Erhöhung des Feuchteanteils zu erreichen, als bei einem feuchteren Futtermittelgemisch. Das gilt umso mehr in Fällen, wo mehrere Futtermittelkomponenten mit jeweils unterschiedlichem Feuchteanteil zu einem Futtermittelgemisch mit einem zusammengesetzten Feuchteanteil vermengt werden.

Bei Überschreiten eines oberen Grenzwerts der gemessenen Feuchtigkeit kann die Befüllmenge von zumindest einer beigemengten Futtermittelkomponente um einen Wert erhöht werden, bei dem die feuchtigkeitsfreie Trockenmasse dieser Futtermittelkomponente im Futtermittelbehälter einem vorgegebenen Zielwert entspricht. Dabei handelt es sich um jene Futtermittelkomponenten, deren Feuchtigkeit in der Regel einer stärkeren Variation der Feuchtigkeit unterworfen ist, beispielsweise Silage- oder Heukomponenten. Durch deren erhöhte Befüllmenge im Futtermittelbehälter wird deren Anteil am Futtermittelgemisch erhöht, um deren Trockenmasse, die durch die zu starke Feuchtigkeit verfälscht wird, an einen gewünschten Zielwert der Aufnahme dieser Futtermittelkomponenten heranzuführen. Die für die Nahrungsaufnahme eines Tieres gewünschte Menge einer Futtermittelkomponente richtet sich nämlich genau genommen nach der feuchtigkeitsfreien Trockenmasse dieser Futtermittelkomponente, also die Masse der Futtermittelkomponente bei Abwesenheit von freiem Wasser. Wird beispielsweise über die Wiegeeinrichtung eine vorgegebene Futtermittelmasse detektiert, sinkt bei zunehmendem Feuchteanteil einer Futtermittelkomponente die verabreichte Trockenmasse dieser Futtermittelkomponente. Die Menge der zusätzlich beizumengenden Futtermittelkomponente zum Erreichen eines vorgegebenen Zielwerts für die zu verabreichende Trockenmasse dieser Futtermittelkomponente in Abhängigkeit von beispielsweise einem gemessenen Feuchteanteil kann ebenfalls über zuvor erstellte Kalibrierkurven ermittelt werden, oder es wird der Einfachheit halber eine Linearisierung vorgenommen, indem beispielsweise bei einer Überschreitung eines Sollwerts des Feuchteanteils um 15% eine zusätzliche Menge der feuchten Futtermittelkomponenten von 15% ihres Ausgangswerts beigemengt wird.

Vorzugsweise wird die Feuchtigkeit des Futtermittelgemisches im Futtermittelbehälter nach dem Befüllen des Futtermittelbehälters und einer ausreichenden Mischdauer, aber noch vor einer Fütterungsfahrt gemessen. Auf diese Weise kann noch vor der Fütterungsfahrt in Abhängigkeit von den gemessenen Feuchtigkeitswerten eine Beimengung von Wasser oder einer der Futtermittelkomponenten vorgenommen werden. Freilich könnte aber auch vorgesehen sein, dass die Feuchtigkeit des Futtermittelgemisches im Futtermittelbehälter nach dem Befüllen des Futtermittelbehälters während einer Fütterungsfahrt gemessen wird, und die in Abhängigkeit vom gemessenen Feuchtigkeitswert vorgenommene Befüllung des Futtermischwagens mit den Futtermittelkomponenten sowie die in Abhängigkeit vom gemessenen Feuchtigkeitswert vorgenommene Beimengung von Wasser in den Futtermischwagen erst bei erneuter Befüllung nach der Fütterungsfahrt erfolgt.

Zudem wird vorgeschlagen, dass zur Festlegung des unteren Grenzwerts während einer Fütterungsfahrt die Höhenverteilung des in einer vorherigen Fütterungsfahrt abgegebenen Futters quer zur Fahrtrichtung des Futtermischwagens und in Fahrtrichtung gesehen vor dem Futtermischwagen mithilfe von Sensoren ermittelt wird. Als Sensoren können beispielsweise zumindest zwei Ultraschallsensoren verwendet werden, von denen ein Sensor in den vom Vieh erreichbaren Futterbereich gerichtet ist, und ein zweiter Sensor in den vom Vieh nicht erreichbaren Futterbereich gerichtet ist. Das Messergebnis ist jeweils ein Abstand zwischen der höchsten Futterlage und dem Sensor, der in eine Höhe der betreffenden Futterlage umgerechnet werden kann. Die Höhenverteilung ergibt sich aus einem Vergleich der gleichzeitig gemessenen Messwerte der beiden Sensoren. Eine kleine Differenz der beiden Messwerte bei gleichzeitig hoher Summe der Messwerte lässt beispielsweise auf eine starke Verstreuung des zuvor ausgebrachten Futters im Futtergang schließen. Eine Anhebung des unteren Grenzwerts oder des Sollwerts der angestrebten Feuchtigkeit kann in diesem Fall Abhilfe schaffen.

Die Erfindung wird im Folgenden anhand eines Ausführungsbeispiels mithilfe der beiliegenden Zeichnungen näher erläutert. Es zeigen hierbei die
Fig. 1 eine schematische Darstellung für eine Ausführungsform einer Anordnung zur Herstellung und Verteilung eines aus Futtermittelkomponenten gebildeten Futtermittelgemisches in einer Futtergasse landwirtschaftlicher Betriebe,
Fig. 2 eine Ansicht einer Ausführungsform eines Futtermischwagens von der Seite gesehen,
Fig. 3 eine Ansicht der Ausführungsform eines Futtermischwagens gemäß der Fig. 2 von der gegenüberliegenden Seite gesehen,
Fig. 4 eine Ansicht einer Ausführungsform eines Futtermischwagens gemäß der Fig. 2 von oben gesehen.

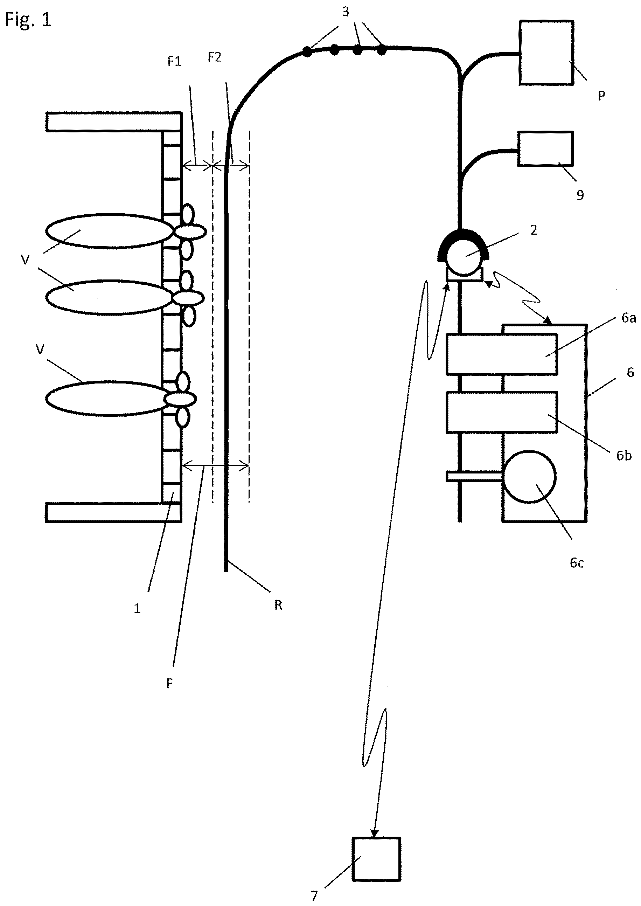

Zunächst wird auf die Fig. 1 Bezug genommen, die eine schematische Darstellung für eine Ausführungsform einer Anordnung zur Herstellung und Verteilung eines aus Futtermittelkomponenten gebildeten Futtermittelgemisches entlang eines Barrens 1 einer Futtergasse F landwirtschaftlicher Betriebe zeigt. Das Vieh V befindet sich auf der anderen Seite des Barrens 1, der aber so ausgeführt ist, dass das Vieh V das im Futtergang F ausgebrachte Futtergemisch erreichen kann, und zwar in einem vom Vieh V erreichbaren, ersten Futterbereich F1 nahe am Barren 1. In der Fig. 1 ist zudem ein zweiter Futterbereich F2 skizziert, der vom Barren 1 weiter entfernt ist und vom Vieh V während der Nahrungsaufnahme nicht mehr erreicht werden kann.

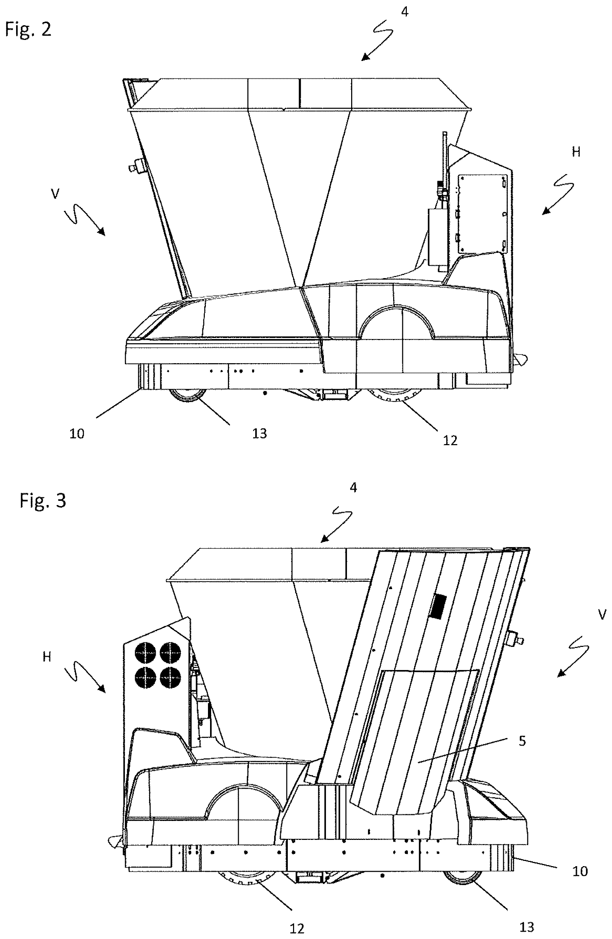

Das Futter wird in einem fahrbaren Futtermischwagen 2 abgemischt und ausgebracht, wobei freilich auch mehrere Futtermischwagen 2 in einem Betrieb vorgesehen sein können, die unterschiedliche Futtergänge F bedienen und über eine externe Steuereinheit koordiniert werden. Die Futtermischwagen 2 sind als autonome Fahrzeuge ausgeführt, die selbsttätig auf dem Stallboden fahren und dabei eine vorprogrammierte Fahrstrecke R absolvieren. Zur Navigation des Futtermischwagens 2 können etwa Markierungsmagneten 3 vorgesehen sein, die entlang der Fahrstrecke R angeordnet sind. Freilich sind auch andere Formen der Navigation, beispielsweise über Kameras und dergleichen, denkbar. In der Fig. 1 sind lediglich beispielhaft vier Markierungsmagneten 3 eingezeichnet. Der Futtermischwagen 2 fährt dabei den Futtergang F nahe am Barren 1 entlang und wirft das im Futtermittelbehälter 4 abgemischte Futter mithilfe einer Abgabevorrichtung 5 (siehe Fig. 3) nahe am Barren 1 aus.

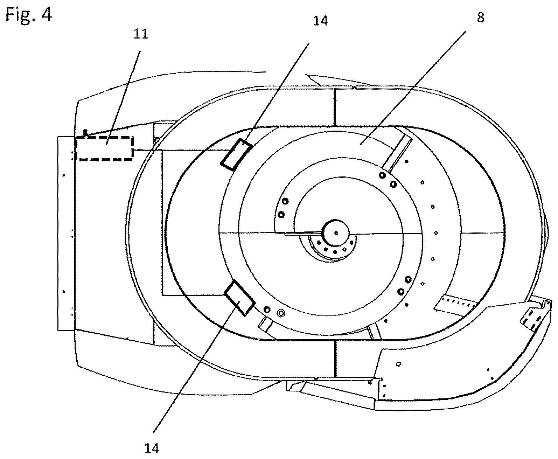

Die Aufnahme der Futtermittelkomponenten erfolgt in einer Befüllstation 6. Dabei werden der Futtermischwagen 2 und die Befüllstation 6 von einer am Futtermischwagen angeordneten Steuereinrichtung 11 gesteuert (siehe Fig. 4). Auf die Steuereinrichtung 11 kann über eine Ausgabeschnittstelle 7 zugegriffen werden, die auf einem PC, einem Tablet oder auch einem Mobilfunkgerät ("Smartphone") installiert sein kann. Über die Steuereinrichtung 11 können insbesondere die Zusammensetzung des Futtermittelgemisches, die Menge, die Fütterungszeiten und die Fahrstrecken R festgelegt werden. Im täglichen Betrieb steuert die Steuereinrichtung 11 den Futtermischwagen 2 je nach programmierter Uhrzeit zur Befüllstation 6, in der die einzelnen Futtermittelkomponenten des Grundfutters, beispielsweise Grassilage, Heu oder Maissilage, sowie die Futtermittelkomponenten des Kraftfutters wie Getreide- oder industriell hergestellte Mischfutterkomponenten, bereitgehalten werden. Der Futtermischwagen 2 fährt beispielsweise einzelne Vorratsbehälter 6a, 6b der Befüllstation 6 ab, die auch als "Ports" bezeichnet werden, und wird dort mit den einzelnen Futtermittelkomponenten befüllt. Zusätzlich kann auch eine Befüllung über Förderbänder oder über Gebläse aus externen Fahrsilos und dergleichen vorgesehen sein. Die Befüllstation 6 umfasst ferner eine Dosiereinrichtung 6c zur Beimengung von Wasser in den Futtermittelbehälter 4. Der Auswurf der Futtermittelkomponenten und die Beimengung von Wasser in den Futtermittelbehälter 4 erfolgt dabei jeweils dosiert mit Befüllmengen, die über die Steuereinrichtung 11 eingestellt werden können. Im gezeigten Ausführungsbeispiel wird der Futtermittelbehälter 4 von oben befüllt.

Im Futtermittelbehälter 4 ist ein Futtermischer 8 (siehe Fig. 4) zur Vermengung der Futtermittelkomponenten zum Futtermittelgemisch angeordnet, der beispielsweise als Mischschnecke mit vertikaler Drehachse ausgeführt ist. Die Mischschnecke kann auch so ausgeführt sein, dass sie das Futter zusätzlich zerkleinert, indem sie entsprechende Schneidorgane aufweist. Sobald alle Futtermittelkomponenten für die betreffende Fütterungsfahrt vom Futtermischwagen 2 aufgenommen wurden, wird mit der vorprogrammierten Fahrstrecke R für diese Fütterungsfahrt begonnen. Gegebenenfalls kann zuvor noch eine befahrbare Ladestation 9 angefahren werden, in der die Batterie des vorzugsweise elektrisch betriebenen Futtermischwagens 2 aufgeladen wird und der Futtermischer 8 während des Ladevorganges bereits für eine gründliche Durchmischung der Futtermittelkomponenten im Futtermittelbehälter 4 sorgt. Sobald die für diese Fütterungsfahrt vorgesehene Futtergasse F erreicht wurde, wird die Abgabevorrichtung 5 des Futtermischwagens 2 betätigt, sodass Futter ausgeworfen wird. Im gezeigten Ausführungsbeispiel handelt es sich bei der Abgabevorrichtung 5 um eine seitlich angeordnete Abgabeklappe, die geöffnet und geschlossen werden kann. Die Auswurfgeschwindigkeit und die Fahrgeschwindigkeit bestimmen dabei die lokal ausgebrachte Futtermenge. Konkret kann dabei etwa so vorgegangen werden, dass die Wiegeeinrichtung am Futtermittelwagen 2 die Veränderung der Futtermenge im Futtermittelbehälter 4 permanent misst und die Steuereinrichtung 11 die Fahrgeschwindigkeit reduziert und/oder die Abgabeklappe weiter öffnet, wenn die gemessene Veränderung der Futtermenge zu klein ist. Wenn die gemessene Veränderung der Futtermenge hingegen zu groß ist, wird die Fahrgeschwindigkeit erhöht und/oder die Abgabeklappe etwas geschlossen.

Das Futter wird dabei gelockert und gut gemischt dem Vieh V angeboten, wobei es möglich ist mehrmals täglich kleinere Futtermengen in unterschiedlicher Zusammensetzung auszubringen, um stets eine frische Futtervorlage zu gewährleisten. Das Ausdosieren kleinerer Futtermengen unterstützt wie bereits erwähnt das natürliche Fressverhalten der Tiere und sorgt damit für eine effizientere Futterverwertung, was für die Tiergesundheit und damit auch die Produktivität des Betriebes förderlich ist.

In weiterer Folge wird auf die Fig. 2 bis 4 Bezug genommen, die mehrere Ansichten einer Ausführungsform eines erfindungsgemäßen Futtermischwagens 2 zeigen. Der Futtermischwagen 2 weist ein Gehäuse mit einem Vorderteil V und einem Heckteil H auf. Im Inneren des Gehäuses befinden sich der Radantrieb für die Räder 12 und/oder Rollen des fahrbaren Gehäuses, der Antrieb für den Futtermischer 8, die Steuereinrichtung 11 sowie eine in der Regel aufladbar ausgeführte Stromversorgung für alle vorgenannten Komponenten. Eine Laufrolle 13 ist im Vorderteil V angeordnet. Durch unterschiedliche Ansteuerung der Räder 12 können unterschiedliche Fahrrichtungen des Gehäuses verwirklicht werden. Die Ansteuerung der Räder 12 wird über die Steuereinrichtung 11 (siehe Fig. 4) eingestellt, die wiederum mit Positionssensoren verbunden ist, die die Position des Gehäuses relativ zu den externen Markierungsmagneten 3 bestimmen, mit einer vorgegebenen Fahrstrecke R vergleichen und entsprechende Steuersignale an die Räder 12 setzen um die vorgegebene Fahrstrecke R beizubehalten.

Die zur Stromversorgung der vorgenannten Komponenten erforderliche Betriebsspannung wird von vorzugsweise aufladbaren Batterien bereitgestellt. Die Steuereinrichtung 11 des Futtermischwagens 2 erkennt dabei über eine Ladekontrolle den Ladezustand der Batterie und steuert rechtzeitig eine Ladestation 9 an. Sobald der Futtermischwagen 2 die Ladestation 9 erreicht hat, koppelt er automatisch an die Ladestation 9 und beginnt mit dem Ladezyklus. Nachdem ein ausreichender Ladezustand der Batterie erreicht wurde, setzt der Futtermischwagen 2 die Fahrt fort. Die Steuereinrichtung 11 des Futtermischwagens 2 ist zudem im gezeigten Ausführungsbeispiel mit einer Ausgabeschnittstelle 7 und über eine drahtlose Signalverbindung mit der Befüllstation 6 zur Steuerung der Befüllung verbunden.

Der Futtermischwagen 2 kann zudem in seinem bodennahen Bereich mit einer Schieberschürze 10 versehen sein, die zuvor ausgebrachtes und vom Vieh V während der Nahrungsaufnahme im Futtergang F verstreutes Futter wieder zum Barren 1 schiebt. Der Futtermischwagen 2 weist zudem in der Regel auch eine Wiegeeinrichtung auf (in den Fig. 1-4 nicht ersichtlich), die die im Futtermittelbehälter 4 vorhandene Futtermenge misst. Nach Absolvierung der Fütterungsfahrt und dem Ausbringen der vorgesehenen Futtermengen kehrt der Futtermischwagen 2 wieder in eine Parkposition P zurück. Über eine Ausgabeschnittstelle 7 der Steuereinrichtung 11 kann der Futtermischwagen 2 eine Vollzugsmeldung oder auch andere Daten wie zum Beispiel zu allfälligen Restmengen an Futter im Futtermittelbehälter 4 übermitteln.

Im Futtermittelbehälter 4 sind zudem zwei Feuchtigkeitssensoren 14 zur Messung der Feuchtigkeit des Futtermittelgemisches angeordnet, die jeweils mit der Steuereinrichtung 11 verbunden sind. Bei den Feuchtigkeitssensoren 14 handelt es sich beispielsweise um Feuchtigkeitssensoren 14, die jeweils die Leitfähigkeit des Futtergemisches messen. Durch die im Futtermittelbehälter 4 angeordneten Feuchtigkeitssensoren 14 kann der Feuchteanteil des Futtermittelgemisches gemessen werden. Der gemessene Wert wird der Steuereinrichtung 11 zur Steuerung der Befüllung übermittelt. Die Feuchtigkeit des Futtermittelgemisches kann auf diese Weise überwacht und in weiterer Folge in einem optimalen Sollbereich gehalten werden. Die beiden Feuchtigkeitssensoren 14 sind an einer Innenwand des Futtermittelbehälters 4 in einer unteren Hälfte des Futtermittelbehälters 4 in derselben Höhe und entlang eines inneren Umfanges der Innenwand verteilt befestigt. Sie messen dadurch an unterschiedlichen Stellen im Futtermittelbehälter 4 die Feuchtigkeit und übermitteln die gemessenen Werte an die Steuereinrichtung 11, wo eine Mittelung der gemessenen Werte vorgenommen wird.

Die gemessenen Werte werden zum Herstellen des Futtermittelgemisches mit optimaler Feuchtigkeit verwendet, indem bei Unterschreiten eines unteren Grenzwerts der gemessenen Feuchtigkeit dem Futtermittelgemisch im Futtermittelbehälter 4 Wasser beigemengt wird, wie oben beschrieben wurde, und bei Überschreiten eines oberen Grenzwerts der gemessenen Feuchtigkeit die Befüllmenge von zumindest einer beigemengten Futtermittelkomponente erhöht wird. Dabei handelt es sich um jene Futtermittelkomponenten, deren Feuchtigkeit in der Regel einer stärkeren Variation der Feuchtigkeit unterworfen ist, beispielsweise Silage- oder Heukomponenten. Durch deren erhöhte Befüllmenge wird deren Anteil am Futtermittelgemisch erhöht, um deren Trockenmasse, die durch die zu starke Feuchtigkeit verfälscht wird, an einen gewünschten Zielwert der Aufnahme dieser Futtermittelkomponenten heranzuführen.

Die Feuchtigkeit des Futtermittelgemisches im Futtermittelbehälter 4 wird beispielsweise nach dem Befüllen des Futtermittelbehälters 4 und einer ausreichenden Mischdauer, aber noch vor einer Fütterungsfahrt gemessen. Auf diese Weise kann noch vor der Fütterungsfahrt in Abhängigkeit von den gemessenen Feuchtigkeitswerten eine Beimengung von Wasser oder einer der Futtermittelkomponenten vorgenommen werden. Freilich könnte aber auch vorgesehen sein die Feuchtigkeit des Futtermittelgemisches im Futtermittelbehälter 4 nach dem Befüllen des Futtermittelbehälters 4 und während einer Fütterungsfahrt zu messen, und die in Abhängigkeit vom gemessenen Feuchtigkeitswert vorgenommene Befüllung des Futtermischwagens 2 mit den Futtermittelkomponenten sowie die in Abhängigkeit vom gemessenen Feuchtigkeitswert vorgenommene Beimengung von Wasser in den Futtermischwagen 2 bei einer darauffolgenden, erneuten Befüllung nach der Fütterungsfahrt vorzunehmen.

Zudem wird vorgeschlagen, dass zur Festlegung eines geeigneten, unteren Grenzwerts während einer Fütterungsfahrt die Höhenverteilung des in einer vorherigen Fütterungsfahrt abgegebenen Futters quer zur Fahrtrichtung des Futtermischwagens 2 und in Fahrtrichtung gesehen vor dem Futtermischwagen 2 mithilfe von Sensoren ermittelt wird. Als Sensoren können beispielsweise zumindest zwei Ultraschallsensoren verwendet werden, von denen ein Sensor in den vom Vieh V erreichbaren Futterbereich F1 gerichtet ist, und ein zweiter Sensor in den vom Vieh V nicht erreichbaren Futterbereich F2 gerichtet ist. Das Messergebnis ist jeweils ein Abstand zwischen der höchsten Futterlage und dem Sensor, der in eine Höhe der betreffenden Futterlage umgerechnet werden kann. Die Höhenverteilung ergibt sich aus einem Vergleich der gleichzeitig gemessenen Messwerte der beiden Sensoren. Eine kleine Differenz der beiden Messwerte für die Futterhöhe des Futters in dem vom Vieh V erreichbaren Futterbereich F1 und in dem vom Vieh V nicht erreichbaren Futterbereich F2 bei gleichzeitig hoher Summe der Messwerte lässt beispielsweise auf eine starke Verstreuung des zuvor ausgebrachten Futters im Futtergang schließen. Eine Anhebung des unteren Grenzwerts oder des Sollwerts der angestrebten Feuchtigkeit kann in diesem Fall Abhilfe schaffen.

Wie bereits ausgeführt wurde, stellt eine optimale Feuchtigkeit eine ausreichende Verklumpung des Futtermittelgemisches sicher, die dem Vieh V das Selektieren erschwert und das Verstreuen des ausgebrachten Futters im Zuge der Futteraufnahme verringert. Auf diese Weise werden die Aufnahme des auf das jeweilige Vieh V hinsichtlich Menge und Zusammensetzung abgestimmten Futtermittels verbessert und die Tiergesundheit und damit auch die Produktivität des Betriebes gefördert.

## Patentansprüche

1. Fahrbarer Futtermischwagen (2) zur Verteilung eines Futtermittelgemisches in landwirtschaftlichen Betrieben mit einem mit Futtermittelkomponenten befüllbaren Futtermittelbehälter (4), in dem ein Futtermischer (8) zur Vermengung der Futtermittelkomponenten zum Futtermittelgemisch angeordnet ist, wobei der Futtermischwagen (2) mit einer Abgabevorrichtung (5) zur Abgabe des Futtermittelgemisches während einer Fütterungsfahrt versehen ist, und eine Steuereinrichtung (11) zur Steuerung der Befüllung mit den Futtermittelkomponenten in einer Befüllstation (6) vorgesehen ist, **dadurch gekennzeichnet, dass** im Futtermittelbehälter (4) zumindest ein Feuchtigkeitssensor (14) zur Messung der Feuchtigkeit des Futtermittelgemisches angeordnet ist, der mit der Steuereinrichtung (11) verbunden ist.

2. Fahrbarer Futtermischwagen (2) nach Anspruch 1, **dadurch gekennzeichnet, dass** der Feuchtigkeitssensor (14) an einer Innenwand des Futtermittelbehälters (4) in einer unteren Hälfte des Futtermittelbehälters (4) befestigt ist.

3. Fahrbarer Futtermischwagen (2) nach Anspruch 1 oder 2, **dadurch gekennzeichnet, dass** zumindest zwei Feuchtigkeitssensoren (14) vorgesehen sind, die an einer Innenwand des Futtermittelbehälters (4) in einer unteren Hälfte des Futtermittelbehälters (4) in derselben Höhe und entlang eines inneren Umfanges der Innenwand verteilt befestigt sind.

4. Anordnung zur Herstellung und Verteilung eines aus Futtermittelkomponenten gebildeten Futtermittelgemisches in landwirtschaftlichen Betrieben mit einem mit den Futtermittelkomponenten befüllbaren und einer Steuereinrichtung (11) versehenen Futtermischwagen (2) nach einem der Ansprüche 1 bis 3, sowie einer Befüllstation (6) zur Befüllung des Futtermischwagens (2) mit den Futtermittelkomponenten, wobei die Steuereinrichtung (11) ausgelegt ist die Befüllung des Futtermischwagens (2) mit den Futtermittelkomponenten sowie die Beimengung von Wasser in den Futtermischwagen (2) in Abhängigkeit von einem im Futtermischwagen (2) gemessenen Feuchtigkeitswert des Futtermittelgemisches zu steuern.

5. Verfahren zum Herstellen eines Futtermittelgemisches aus Futtermittelkomponenten in einem mit den Futtermittelkomponenten befüllbaren Futtermittelbehälter (4) eines fahrbaren und zur Abgabe des Futtermittelgemisches während einer Fütterungsfahrt ausgelegten Futtermischwagens (2), **dadurch gekennzeichnet, dass** die Feuchtigkeit einer Futtermittelkomponente oder des Futtermittelgemisches gemessen wird, und bei Unterschreiten eines unteren Grenzwerts der gemessenen Feuchtigkeit Wasser der Futtermittelkomponente oder dem Futtermittelgemisch beigemengt wird.

6. Verfahren nach Anspruch 5, **dadurch gekennzeichnet, dass** bei Überschreiten eines oberen Grenzwerts der gemessenen Feuchtigkeit die Befüllmenge von zumindest einer beigemengten Futtermittelkomponente um einen Wert erhöht wird, bei dem die feuchtigkeitsfreie Trockenmasse dieser Futtermittelkomponente einem vorgegebenen Zielwert entspricht.

7. Verfahren nach Anspruch 5 oder 6, **dadurch gekennzeichnet, dass** zur Festlegung des unteren Grenzwerts während einer Fütterungsfahrt die Höhenverteilung des in einer vorherigen Fütterungsfahrt abgegebenen Futters quer zur Fahrtrichtung des Futtermischwagens (2) und in Fahrtrichtung gesehen vor dem Futtermischwagen (2) mithilfe von Sensoren ermittelt wird.
